# EUROPÄISCHE PATENTSCHRIFT

(11) **EP 0 808 302 B1**
(45) Veröffentlichungstag und Bekanntmachung des Hinweises auf die Patenterteilung: **05.04.2000**
(21) Anmeldenummer: 96901746.6
(22) Anmeldetag: 20.01.1996
(51) Int. Cl.: C07C 253/34, C07C 255/43

(54) **VERFAHREN ZUR RACEMATTRENNUNG VON 2-ARYL-2-ALKYL-OMEGA-ALKYLAMINO-ALKANNITRILEN**
2-ARYL-2-ALKYL-OMEGA-ALKYLAMINOALKANE NITRILE RACEMATE SEPARATION PROCESS
PROCEDE DE SEPARATION DE RACEMATES DE NITRILES DE 2-ARYL-2-ALKYL-OMEGA-ALKYLAMINOALCANES

(30) Priorität: 31.01.1995 DE 19502967
(43) Veröffentlichungstag der Anmeldung: 26.11.1997
(73) Patentinhaber: BASF AKTIENGESELLSCHAFT, 67056 Ludwigshafen (DE)
(72) Erfinder: KARL, Ulrich, D-67061 Ludwigshafen (DE); SEITZ, Werner, D-68723 Plankstadt (DE)
(86) Internationale Anmeldenummer: EP9600236
(87) Internationale Veröffentlichungsnummer: WO9623764

(56) Entgegenhaltungen:
- EP-A- 0 357 565
- WO-A-95/09150
- DE-A- 2 059 985

## Beschreibung

An der Phenylgruppe unterschiedlich substituierte sowie mit einer basischen Seitenkette versehene Phenylacetonitrile haben als wirksame Arzneimittelwirkstoffe Anwendung gefunden. So haben sich von den in DE 11 54 810 beschriebenen basisch substituierten Phenylacetonitrilen der Formel II Verapamil (II, R=H) und Gallopamil (II, R=OCH₃) in der Behandlung der koronaren Herzkrankheit und des Bluthochdrucks bewährt.

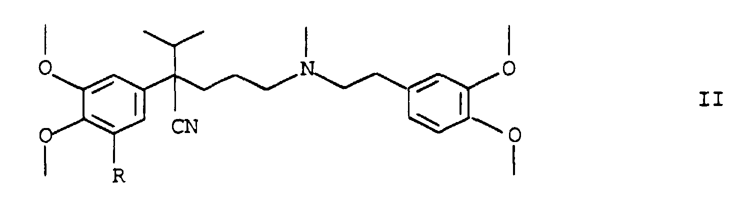

EP 0 271 013 beschreibt Phenylacetonitrile, die eine aliphatische Seitenkette anstelle des Dimethoxyphenylethyl-Rests am Stickstoff tragen, als Wirkstoffe gegen Herzkreislauferkrankungen und asthmatische Erkrankungen.

Die substituierten Phenylacetonitrile weisen ein chirales Kohlenstoffatom auf, so daß sie bei Abwesenheit weiterer Chiralitätszentren zwei Enantiomere (optische Antipoden) bilden. Im Rahmen der üblichen chemischen Synthesen aus achiralen Ausgangsstoffen entstehen beide Enantiomeren in gleichen Mengen, so daß ein Racemat vorliegt.

Von Verapamil und Gallopamil ist bekannt, daß sich die Enantiomeren hinsichtlich ihrer Pharmakodynamik und -kinetik quantitativ deutlich unterscheiden (M. Raschack, Naunyn-Schmiedeberg's Arch. Pharmacol. 1976, 294, 285; M. Eichelbaum u.a., Br. 7. Clin. Pharmacol. 1984, 17, 453). So ist das linksdrehende Enantiomer viel stärker koronardilatatorisch wirksam, während der rechtsdrehende Antipode als Resistenzbrecher in die Malaria- bzw. Tumortherapie Eingang fand.

Daher ist die Herstellung optisch aktiver Phenylacetonitrile von großer Bedeutung.

Allerdings ist diese Herstellung sehr schwierig: Beim Einsatz von chiralen Katalysatoren bei der Synthese von Verapamil konnte nur ein Enantiomerenüberschuß von maximal 10 %, d.h, ein Verhältnis der Enantiomeren von 55 : 45, erreicht werden (H. Brunner und H. Zintl, Monatsh. Chemie 122, 841 - 848 [1991]). Auch andere chirale Katalysatoren liefern nur völlig unzureichende Enantiomerenüberschüsse.

Die von L.J. Theodore und W.L. Nelson (J. Org. Chem. 52, 1309 - 1315 (1987)) beschriebene stereospezifische Synthese der Enantiomere von Verapamil und Gallopamil geht von den stereoisomeren Milchsäuren aus und ist aufgrund der zahlreichen Reaktionsstufen und komplizierten chemischen Verwandlungen von Funktionalitäten nicht im technischen Maßstab durchführbar.

Häufiger wurden Racematspaltungsmethoden publiziert. Ein Sonderfall liegt in WO 92/07821 vor, wo die Racematspaltung des Phenylacetonitril-Teils durch Verknüpfung mit optisch aktivem β-Methylaminotetralin, welches Bestandteil des Wirkstoffs wird, und fraktionierte Kristallisation der Diastereomere erfolgt:

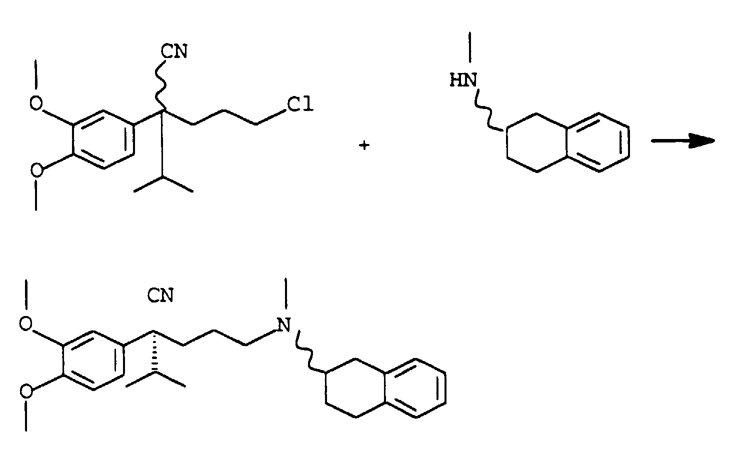

Vielseitiger anwendbar ist die intermediäre Bildung diastereomerer Salze, die sich mittels Kristallisation oder Chromatographie auftrennen lassen, und Freisetzung des gewünschten optisch aktiven Produkts aus dem Salz.

Die Schwierigkeit der Racematspaltung zeigt sich daran, daß es lange Zeit nicht gelang, das Endprodukt selbst, d.h. den Wirkstoff oder eine seiner basischen Vorstufen, die bei den Syntheseverfahren üblicherweise durchschritten werden, aufzuspalten, sondern daß die Carboxyderivate III bzw. IV, deren Umsetzung zum gewünschten Produkt Verapamil zusätzliche Syntheseschritte erforderlich macht, mit chiralen Basen racematgespalten wurden.

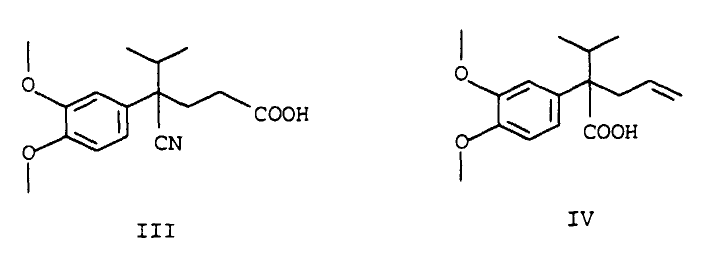

Zur Aufspaltung von III wird Brucin verwendet (DE 2059 985), was aufgrund der extremen Toxizität und des hohen Preises von Brucin nachteilig ist. IV kann zwar mit Chinchonidin gespalten werden, muß aber an zwei Funktionalitäten modifiziert werden, um zum gewünschten Produkt zu gelangen, was technisch schwierig durchführbar ist (H. Ramuz, Helv. Chim. Acta 58, 2050 - 2060 [1975]).

Die in EP 0271 013 in den Beispielen 6 und 7 verwendeten chiralen Vorstufen V und VI wurden nach der in DE 2059 985 beschriebenen Methode hergestellt.

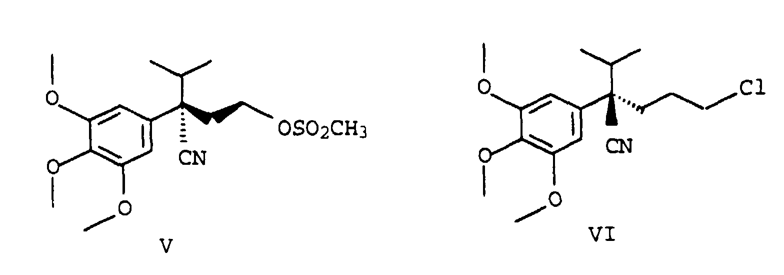

Erst spät wurden Verfahren zur Racematspaltung der freien Base von Verapamil beschrieben. In DE-OS 3 723 684 wird (R)- bzw. (S)-2,2'-(1,1'-Binaphthyl)phosphorsäure als Spaltsäure verwendet, die jedoch äußerst aufwendig in der Herstellung ist und nicht in technischen Mengen zur Verfügung steht. DE-OS 4 203 547 benutzt ein bis zwei Äquivalente O,O'-Dibenzoyl- oder O,O'-Di-p-toluolweinsäure, die sehr teuer sind und bei der Freisetzung des Verapamils leicht hydrolysiert werden und somit verloren gehen.

Insbesondere in Anbetracht der Feststellung von H. Ramuz (Helv. Chim. Acta 58, 2050 [1975], daß sich Camphersulfonsäure nicht zur Racematspaltung von Verapamil eignet, war es überraschend, daß die basisch substituierten Phenylacetonitrile mit Camphersulfonsäure leicht zu hochenantiomerenreinen optischen Antipoden aufgespalten werden können.

Gegenstand der Erfindung ist ein Verfahren zur Racemattrennung von 2-Aryl-2-alkylen-ω-alkylamino-alkannitrilen der Formel I 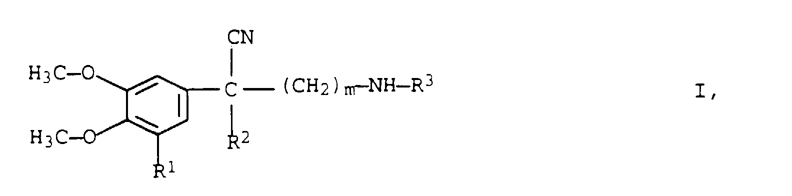 worin
- R¹: Wasserstoff oder Methoxy ist,
- R² und R³,: die gleich oder verschieden sind, C₁₋₄-Alkyl bedeuten und
- m: die Zahl 2 oder 3 ist,
in an sich bekannter Weise, dadurch gekennzeichnet, daß man als Spaltungsreagen optisch aktive Camphersulfonsäure verwendet.

Als C₁₋₄-Alkylgruppen seien folgende genannt: Methyl, Ethyl, n-Propyl, Isopropyl, Cyclopropyl, n-Butyl, Isobutyl, sec-Butyl, tert.-Butyl.

Das erfindungsgemäße Verfahren wird vorzugsweise wie folgt durchgeführt: 1 Äquivalent racemisches I wird in einem Alkohol wie Methanol, Ethanol, n-Propanol, iso-Propanol, Glykol, Diglykol oder einer Mischung der Alkohole oder einer Mischung eines Alkohols mit Aceton oder Essigester (vorzugsweise Isopropanol) vorgelegt, und es werden 0,5 bis 1 (vorzugsweise 0,75) Äquivalent Camphersulfonsäuremonohydrat zugegeben. Danach wird auf 40°C bis 100°C bzw. bis zum Siedepunkt des verwendeten Lösungsmittels erhitzt (vorzugsweise 60°C). Beim Abkühlen kristallisiert das diastereomere Camphersulfonat der Verbindung I aus:
(S)-(+)-Camphersulfonsäure fällt als Salz mit (S)-I,
(R)-(-)-Camphersulfonsäure mit (R)-I aus.

Das Salz wird einmal vorzugsweise aus dem bereits verwendeten Lösemittel bzw. Lösemittelgemisch umkristallisiert.

Zur Freisetzung des optisch aktiven I wird das Salz in verdünnter alkalischer Lösung (z.B. Natronlauge, Kalilauge, Kaliumcarbonat-Lösung) gelöst und mit einem mit Wasser nicht mischbaren Lösungsmittel extrahiert (z.B. Essigester, MTBE (= Methyl-tert.-butylether) Dichlormethan). Die optisch aktive Verbindung I kann aus diesen Lösungen ausgefällt oder direkt darin weiter umgesetzt werden.

Das erfindungsgemäße Verfahren ist äußerst günstig, weil Camphersulfonsäure sehr billig ist und auch in technischen Mengen erhältlich ist und weil die erhaltenen Zwischenprodukte der Formel I die Herstellung zahlreicher Wirkstoffe wie z.B. Verapamil, Gallopamil und der in EP 0 271 013 beschriebenen Verbindungen erlauben.

Die für das erfindungsgemäße Verfahren verwendeten racemischen Ausgangsverbindungen der Formel VI können nach folgendem Schema hergestellt werden:

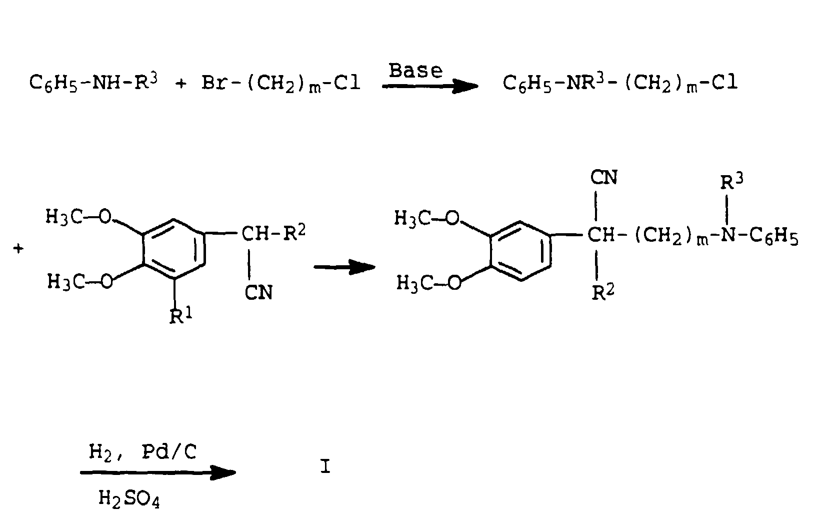

Die folgenden Beispiele sollen das erfindungsgemäße Verfahren verdeutlichen.

### Beispiel 1

### (S)-(-)-5-(N-Methylamino)-2-(1-methylethyl)-2-(3,4,5-trimethoxyphenyl)pentannitril [I, R¹ = OCH₃, R² = CH(CH₃)₂, R³ = CH₃]

95,4 kg (297,8 mol) racemisches 5-(N-Methylamino)-2-(1-methylethyl)-2-(3,4,5-trimethoxyphenyl)- pentannitril wurden zusammen mit 55,9 kg (223,4 mol) (S)-(+)-Campher-10-sulfonsäuremonohydrat in 200 l Isopropanol vorgelegt.

Es wurde auf 60°C Innentemperatur erhitzt, wobei man eine klare Lösung erhielt. Dann wurde langsam abgekühlt. Nach Animpfen mit dem Salz bei 30 bis 35°C begann die Kristallisation. Unter Rühren wurde auf 20°C abgekühlt. Der Niederschlag wurde abfiltriert, mit 50 l Isopropanol gewaschen und aus 300 l Isopropanol umkristallisiert.

Das getrocknete diastereomere Salz wurde in 300 l Wasser mit 30 kg 50 %iger Natronlauge gelöst und zweimal mit je 100 l Methyl-t-butylether extrahiert.
Ausbeute: 30,9 kg (96,5 mol) ≙ 66 % d.Th.
[α]_{D}²⁰ - 17,8° (c = 1, Toluol).

Das Produkt liegt als Öl vor. Das Hydrochlorid schmilzt bei 192 bis 194°C,
[α]_{D}²⁰ - 9,0° (c = 1, Ethanol abs.).

### Beispiel 2

### (R)-(+)-5-N-Methylamino-2-(1-methylethyl)-2-(3,4,5-trimethoxyphenyl)pentannitril

Analog Beispiel 1 erhält man bei Verwendung von (R)-(-)-Campher-10-sulfonsäuremonohydrat als Spaltsäure das rechtsdrehende Enantiomere. Die reine Base zeigt einen spezifischen Drehwert von [α]_{D}²⁰ = +17,8° (c = 10 mg/ml, Toluol).

Das Hydrochlorid schmilzt bei 192 bis 194°C, [α]_{D}²⁰ = 9,1° (c = 1, Ethanol abs.).

### Beispiel 3

### (S)- (-)-5-(N-Methylamino)-2-(1-methylethyl)-2- (3,4-dimethoxyphenyl)pentannitril

Die Substanz wurde analog Beispiel 1 hergestellt. Der spezifische Drehwert der freien Base beträgt [α]_{D}²⁰ = -5,0° (c = 1, Ethanol abs.). Das Hydrochlorid schmilzt bei 172 bis 173°C.

### Beispiel 4

### (R)-(+)-5-N-Methylamino-2-(1-methylethyl)-2-(3,4-dimethoxyphenyl)pentannitril

Die Substanz wurde analog Beispiel 2 hergestellt. Die Base liegt als Öl vor und zeigt einen spezifischen Drehwert von [α]_{D}²⁰ = +5° (c = 1, Ethanol abs.). Das Hydrochlorid schmilzt bei 172 bis 175°C.

## Patentansprüche

1. Verfahren zur Racemattrennung von 2-Aryl-2-alkyl-ω-alkylaminoalkannitrilen der Formel I 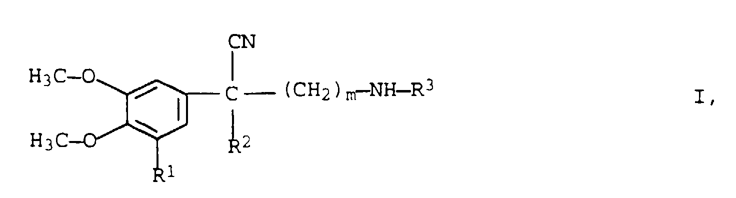 worin
R¹ Wasserstoff oder Methoxy ist,
R² und R³, die gleich oder verschieden sind, C₁₋₄-Alkyl bedeuten und
m die Zahl 2 oder 3 ist,
in an sich bekannter Weise, dadurch gekennzeichnet, daß man als Spaltungsreagenz optisch aktive Camphersulfonsäure verwendet.

## Claims

1. A process for resolving the racemates of 2-aryl-2-alkyl-ω-alkylaminoalkanenitriles of the formula I 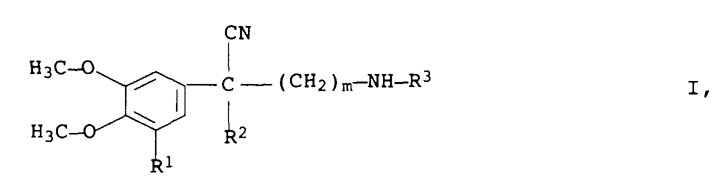 where
R¹ is hydrogen or methoxy,
R² and R³, which are identical or different, are C₁₋₄-alkyl, and
m is 2 or 3,
in a conventional way, wherein optically active camphorsulfonic acid is used as resolving reagent.

## Revendications

1. Procédé pour résoudre les racémates des 2-aryl-2-alkyl-omégaalkylaminoalcanenitriles de formule I 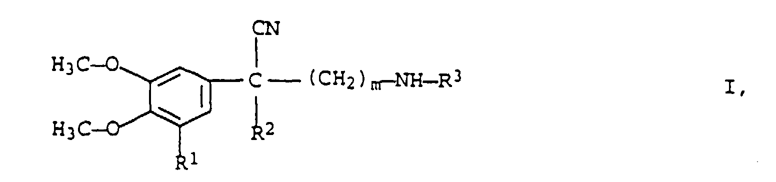 dans laquelle
R¹ représente l'hydrogène ou un groupe méthoxy,
R² et R³, ayant des significations identiques ou différentes, représentent chacun un groupe alkyle en C1-C4 et
m est égal à 2 ou 3,
de manière connue en soi, caractérisé par le fait que l'on utilise, en tant que réactif de résolution, l'acide camphosulfonique à l'état optiquement actif.
